# EUROPÄISCHE PATENTSCHRIFT

(11) **EP 0 489 965 B1**
(45) Veröffentlichungstag und Bekanntmachung des Hinweises auf die Patenterteilung: **04.01.1995**
(21) Anmeldenummer: 90124185.1
(22) Anmeldetag: 14.12.1990
(51) Int. Cl.: A61N 1/05

(54) **Herzschrittmacherleitung mit Schraubwendel**
Pacemaker lead with helix
Conduit de stimulateur cardiaque muni d'une hélice

(43) Veröffentlichungstag der Anmeldung: 17.06.1992
(73) Patentinhaber: Osypka, Peter, Dr. Ing., 79639 Grenzach-Wyhlen (DE)
(72) Erfinder: Osypka, Peter, Dr. Ing., 79639 Grenzach-Wyhlen (DE)
(74) Vertreter: Patent- und Rechtsanwaltssozietät, Schmitt, Maucher & Börjes

(56) Entgegenhaltungen:
- EP-A- 0 000 725
- EP-A- 0 337 035
- GB-A- 2 067 411

## Beschreibung

Die Erfindung betrifft eine implantierbare Herzschrittmacherleitung mit einem isolierenden Schlauch und mit einer zum Fixieren im Inneren des Herzens dienenden, in dieses einschraubbaren Schraubwendel, deren äußerstes und vorderstes Ende spitz und scharf ist, die gegenüber dem Schlauchende einen festen Überstand hat und eine abziehbare Schutzvorrichtung aufweist.

Derartige Herzschrittmacherleitungen sind als implantierbare Schraubelektroden seit vielen Jahren bekannt. Aus der DE-A-25 39 553 ist dabei eine Herzschrittmacherleitung der eingangs erwähnten Art mit starr und feststehender Schraubwendel bekannt, die beim Implantieren und Einführen durch ein Blutgefäß in das Herz Schwierigkeiten dadurch machen kann, daß sich vor allem die spitze der Schraubwendel in der Vene oder auch in der Herzklappe verfangen oder verhaken kann oder zu Verletzungen in diesen Bereichen führen kann. Vor allem relativ enge Richtungsänderungen der Blutgefäße bedeuten entsprechende Gefahren.

Es ist deshalb auch schon bekannt geworden, die Schraubwendet während des Einführens innerhalb des isolierenden Schlauches vorzusehen und zum Fixieren mit Hilfe eines Gewindeganges aus dem Schlauch herauszuschrauben. Beispiele dafür sind die US-A-4 217 913 oder die US-A-4 106 512. Der Vorteil dieser Lösung besteht darin, daß die Gefahren während des Einführens durch die Blutgefäße und in das Herz praktisch ausgeschlossen sind. Dieser Vorteil wird jedoch dadurch erkauft, daß ein aufwendiger und auch recht präziser Mechanismus vorhanden sein muß, der das Herausschrauben oder Vorschieben der Schraubwendel aus dem isolierenden Schlauch ermöglicht und ausschließlich während des Einführrens der Schraubwendel bis in die Herzkammer notwendig ist. Für einen praktisch einmaligen Gebrauch muß also ein relativ großer technischer Aufwand betrieben werden. Ist die Schraubwendel in der Herzkammer fixiert, wird dieser Mechanismus nicht mehr benötigt, bleibt aber implantiert. Dabei ergibt sich aufgrund dieses Schraubmechanismus zwangeläufig ein größerer Elektrodenkopf als bei Herzschrittmacherleitungen mit feststehender und von vorneherein gegenüber dem Schlauchende überstehender Schraubwendel. Ein vergrößerter Elektrodenkopf hat jedoch wiederum eine größere mechanische Irritation an der Herzinnenwand zur Folge, was zu einer Reizschwellenerhöhung führen kann. Darüber hinaus ist eine Abdichtung des Austrittes der Schraubwendel aus der Herzschrittmacherleitung gegen ein Eindringen von Blut in diese schwierig und nicht immer gegeben.

Um einen solchen Schraubmechanismus zu vermeiden, ist aus der EP-A-219 608 eine Lösung bekanntgeworden, bei welcher ein Führungsrohr die Herzschrittmacherleitung einschließlich der Schraubwendel umschließt, solange die Herzschrittmacherleitung implantiert wird. Anschließend kann das Führungsrohr entfernt werden, weil es eine über seine Länge reichende Sollreißlinie hat, so daß dadurch auch die Schraubwendel für das Eindrehen freigegeben wird. Dadurch können die Vorteile einer Schraubwendel - sofortige einfache und gute Befestigung nach dem Implantieren und Möglichkeit für den Patienten, sich sofort ungehindert zu bewegen - erreicht werden, ohne einen Schraubmechanismus zu benötigen und gleichzeitig ohne Gefahr einer Verhakung oder Verletzung während des Implantierens. Es muß allerdings ein nur einmal verwendbarer, herausziehbarer und mit Sollreißlinie versehener Führungsschlauch vorgesehen werden.

Aus der Praxis ist eine Herzschrittmacherleitung der eingangs erwähnten Art unter dem Handelsnamen "sweat tip" der Firma CPI, Cardiac Pacemaker Incorp., 4100 Helmlein, Avenue North, St. Paul Minnesota 55112, USA, bekannt. Die Schraubwendel ist dabei durch eine sich im Blut auflösende Substanz geschützt, so daß sie während des Implantierens ebenfalls keine Verletzungsgefahr für die Gefäßwände oder die Herzklappe bedeutet und sich nicht in engen Biegungen oder beim Eintritt in das Herz verhaken kann, jedoch besteht dabei das Problem, daß der Arzt eine ganz genau vorgegebene Zeit warten muß, bis sich das die Schraubwendel umschließende Material aufgelöst hat, was sehr ungenau ist und vor allem bei einer schwierigen Implantation unter Umständen zu unzutreffenden Zeiten und Ergebnissen führt. Vor allem könnte bei einer schwierigen Implantation das Schutzmaterial zu früh aufgelöst sein und dann doch die befürchtete Verletzungsgefahr auftreten. Andererseits ist es ungünstig, wenn eine bestimmte Zeit gewartet werden muß, bevor die Implantation beendet werden kann.

Es besteht deshalb die Aufgabe, eine Herzschrittmacherleitung der eingangs erwähnten Art zu schaffen, bei der also die Vorteile einer Schraubwendel und deren starre und feste Anordnung mit Überstand gegenüber dem isolierenden Schlauch möglich ist, ohne daß es beim Einführen zu einer Verletzungsgefahr oder Gefahr des Verhakens kommt und ohne daß beim Implantieren eine gewisse Zeit abgewartet werden muß.

Die überraschende Lösung dieser scheinbar widersprüchlichen Aufgabe besteht darin, daß die Schraubwendel einen an ihrer Windung anliegenden und wenigstens die Spitze umkleidenden Überzug aufweist, dessen Innen- und Außenkontur der Außenkontur des von ihr umkleideten Bereiches des die Schaubwendel bildenden Drahtes etwa entspricht, daß an dem Überzug ein zum proximalen Ende der Herzschrittmacherleitung führendes Zugelement angreift und daß der Überzug mittels des Zugelementes von der Spitze der Schraubwendel abziehbar ist.

Im Gegensatz zu einer die gesamte Schraubwendel von außen umschließenden Verlängerung des Schutzschlauches oder einer sonstigen äußeren Umhüllung ist also ein die Windung und die Spitze der Wendel unmittelbar und formschlüssig umschließender Überzug vorgesehen, der ein Verhaken und Verletzen beim Einführen der Herzschrittmacherleitung und ihrer Schraubwendel in das Herz verhindert, aber eine aus dem isolierenden Schlauch herausschraubbare Schraubwendel mit dem entsprechenden Schraubmechanismus überflüssig macht. Dieser bezüglich Innen- und Außenkontur etwa der Schraubwendel und ihrer Spitze entsprechende Überzug kann also weitgehend formschlüssig an dem vordersten Wendelabschnitt oder gegebenenfalls auch der gesamten Schraubwendel direkt anliegen und nach dem Einführen in das Herz vor dem Einschrauben mit Hilfe des Zugelementes abgezogen und durch das Blutgefäß auch völlig zurückgezogen werden. Somit ergeben sich die Vorteile einer starr und gegenüber dem isolierenden Schlauch mit festem Abstand überstehenden Schraubwendel, d.h. ein Schraubmechanismus und eine Vergrößerung des Elektrodenkopfes werden vermieden, ohne daß die dadurch bisher verursachten Gefahren beim Einführen in Kauf genommen werden müssen. Auch ein den gesamten Schraubwendelbereich praktisch haubenförmig umschließendes und ausfüllendes, sich auflösendes Material ist dadurch überflüssig.

Besonders zweckmäßig ist es dabei, wenn der Überzug kappenartig ausgebildet und in Gebrauchsstellung als Windung oder Windungsabschnitt entsprechend der Krümmung der Windung der Schraubwendel geformt ist. Der Überzug stellt also gewissermaßen eine Hohl-Wendel entsprechend der Schraubwendel dar und zwar über den Längenbereich der Schraubwendel, der von ihm umschlossen ist. Dabei muß in vorteilhafter Weise dieser wendelförmige Überzug nicht unbedingt eine entsprechende Eigensteifigkeit haben, da er seine Wendelform dadurch erhalten kann, daß er auf die Schraubwendel aufgeschoben oder aufgewickelt oder aufgebracht ist.

Das Zugelement kann ein Faden oder Draht sein, der parallel zu der Herzschrittmacherleitung oder durch einen in deren Innerem befindlichen Kanal geführt sein kann. Somit kann dieses Zugelement problemlos zusammen mit der Herzschrittmacherleitung zunächst eingeführt werden und dann dazu dienen, den Überzug von der Schraubwendel ab- und aus dem Herzen sowie den Gefäßen zurückzuziehen.

Eine zweckmäßige Ausgestaltung der Erfindung kann darin bestehen, daß der Überzug ein am distalen Ende geschlossener Schutzschlauch ist, der die Spitze und wenigstens etwa ein Viertel bis ein Drittel der distalen letzten Windung der Schraubwendel, gegebenenfalls die halbe oder die gesamte Wendel umschließt, und daß das Zugelement vorzugsweise an dem die Spitze umkleidenden Ende des Schlauches angreift. Ein solcher Schutzschlauch eignet sich gut als Überzug, weil er einfach auf das Wendel-Ende aufschiebbar, gegebenenfalls aufstülpbar ist, unter Umständen dabei sogar etwas aufgeweitet werden kann, also reibschlüssig und formschlüssig befestigt sein kann, dabei problemlos die entsprechende Kontur und Form der Wendel-Spitze und des von ihm umschlossenen Windungsbereiches annehmen kann und einen guten Schutz beim Implantieren der Herzschrittmacherleitung darstellt, aber anschließend auch einfach wieder abgezogen werden kann und durch die Herzschrittmacherleitung oder sogar neben dieser durch das Blutgefäß herausgezogen werden kann.

Es ist aber auch möglich, daß der Überzug eine eng gewickelte Wendel aus einem Kunststoff-Filament oder aus Metalldraht ist, die über die Spitze der Einschraubbwendel ragt. Eine enggewickelte Wendel ist einerseits ein guter Schutz und eine gute Abdeckung einer Spitze und kann andererseits ebenfalls bequem an die gekrümmte Form der Einschraubwendel im Bereich von deren Spitze angepaßt werden.

Dabei kann der Durchmesser des Überzuges - sei es eine Kappe oder ein Schlauch, sei es eine enggewickelte Wendel - im Bereich der Spitze der Schraubwendel deren Durchmesserverkleinerung folgend abnehmen und bis über diese Spitze reichen und dort einem gegenüber dem Querschnitt der die Schraubwendel bildenden Windung erheblich verminderten Durchmesser haben. Dadurch wird erreicht, daß auch die Spitze der Schraubwendel derart umschlossen ist, daß sie nicht aus dem Überzug vorstehen oder aus ihm austreten kann, selbst wenn beim Einführen der Herzschrittmacherleitung entsprechende Schubkräfte auf den Überzug ausgeübt werden.

Vor allem die Verwendung einer enggewickelten Wendel als Überzug gestattet eine Ausgestaltung der Erfindung dahingehend, daß der zum Abziehen des Überzuges dienende Faden einstückig mit der Überzug-Wendel verbunden ist. Der Kunststoffaden oder der Draht, aus welchem der Überzug gewickelt ist, kann also vom Ende des Überzuges aus unmittelbar zum proximalen Ende der Herzschrittmacherleitung fortgesetzt sein und dazu dienen, nach dem Einführen der Herzschrittmacherleitung und ihrer Schraubwendel in das Herz den Überzug abzuziehen.

Dabei kann der zum Zurückziehen dienende Faden oder Draht von dem im Bereich der Spitze der Wendel liegenden Ende der Überzug-Wendel ausgehen. Einerseits dient er dabei dann als zusätzlicher Schutz gegen einen Austritt der Spitze der Schraubwendel beim Implantieren der Herzschrittmacherleitung und andererseits ergibt sich so ein bequemes Abziehen von dem Ende der Schraubwendel, wobei unter Umständen die Windungen der Überzug-Wendel abgewickelt oder geradegezogen werden können, so daß der Schutz-Überzug sehr einfach entfernt werden kann.

Es besteht aber auch die Möglichkeit, daß das Zugelement an dem proximalen Ende der Überzug-Wendel angreift. Auch dann kann durch eine entsprechende Zugkraft eine Windung der Wendel nach der anderen abgewickelt und so der gesamte Überzug abgezogen werden.

Das - fadenförmige oder drahtförmige - Zugelement kann an dem distalen Ende der Zuleitung zum Elektrodenkopf durch eine enge Öffnung in eine zu dem Elektrodenkopf gehörende Kammer eintreten und durch eine weitere, diese Kammer begrenzende Wandung in das Innere der Zuleitung und in den im Inneren der Zuleitung befindlichen Kanal austreten. Dadurch wird das Zugelement insbesondere im Bereich des Elektrodenkopfes in radialer Richtung gut festgelegt und in axialer Richtung geführt und erlaubt die Anordnung einer solchen Kammer, in welcher beispielsweise auch ein Medikament untergebracht sein kann, welches im Herzen aus dieser Kammer austreten soll. Die Öffnung der Kammer erhält so eine Doppelfunktion, indem sie einerseits zur Führung des Zugelementes und andererseits zum Austritt des Medikamentes dient. Dieses in der Kammer enthaltene Medikament kann dabei durch die distale Eintrittsöffnung des Zugelementes allmählich austreten. Gleichzeitig dichten die stirnseitigen Wandungen mit den Durchtritten für das Zugelement den inneren Kanal der Herzschrittmacherzuleitung gegen den Eintritt von Blut ab, wenn die Durchtritte für das Zugelement so eng und dicht sind, daß das Zugelement reibschlüssig inihnen verläuft. Insgesamt ergibt sich eine Herzschrittmacherleitung mit den Vorteilen einer starren Schraubwendel, deren scharfe Spitze dennoch beim transvenösen Einführen keine Verletzungen hervorrufen und keine Verhakungen verursachen kann, weil vor allem diese Spitze bis zum Einführen in das Herz und vor dem endgültigen Einschrauben von einem Überzug eng und fest umschlossen ist, welcher Überzug dann mit Hilfe des Zugelementes ab- und zurückgezogen werden kann.

Nachstehend sind Ausführungsbeispiele der Erfindung anhand der Zeichnung näher beschrieben.

Es zeigt in zum Teil schematisierter Darstellung:

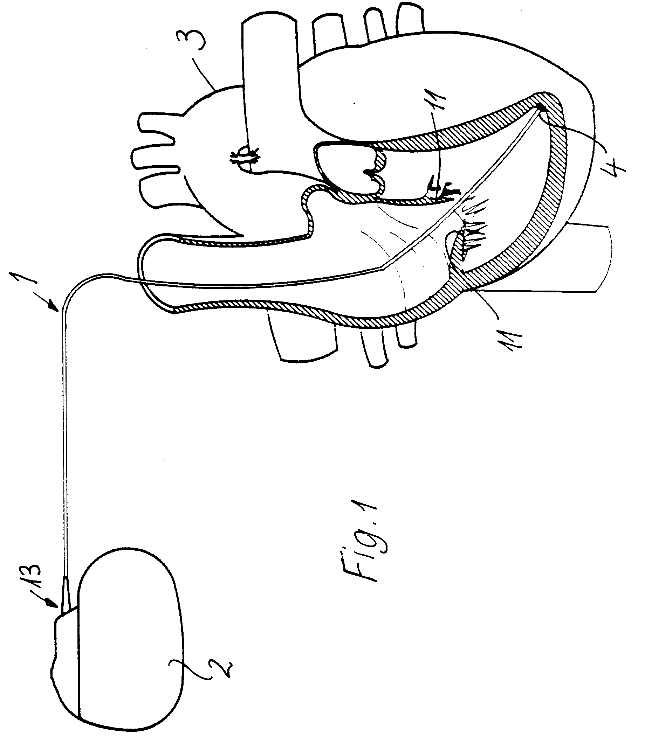

- Fig. 1: eine Herzschrittmacherleitung, die von einem Schrittmacher durch nicht näher dargestellt Blutgefäße bis ins Innere des Herzens führt,
- Fig. 2: im Längsschnitt das vordere Ende und den Elektrodenkopf der Herzschrittmacherleitung mit einer gegenüber isolierenden Schlauch der Zuleitung überstehenden, festen Schraubwendel, die zum Implantieren auf dem Ende ihrer Windungen einen schlauchförmigen oder kappenförmigen Überzug hat,
- Fig. 3: eine der Fig. 2 entsprechende Darstellung und Ausführungsform, bei welcher der Überzug der Spitze der Schraubwendel und des dieser benachbarten Wendelbereiches eine enggewickelte Wendel ist, wobei in den beiden vorstehend genannten Fällen ein Zugelement zum Abziehen des Überzuges durch das Innere der Herzschrittmacherleitung geführt ist,
- Fig. 4: eine der Fig.2 entsprechende Ausführungsform, bei welcher das Zugelement zum Abziehen des Überzuges der Spitze der Schraubwendel parallel zu der Herzschrittmacherleitung geführt ist,
- Fig. 5: eine der Fig. 3 entsprechende Ausführungsform, bei welcher das Zugelement zum Abziehen des wendelförmigen Überzuges parallel zu der Herzschrittmacherleitung an deren Außenseite geführt ist,
- Fig. 6: eine Ausführungsform, bei welcher als Überzug wiederum eine enggewickelte Wendel dient, bei welcher das Zugelement von deren der Spitze abgewandtem Ende ausgeht und einstückig damit verbunden ist und durch das Innere der Zuleitung - gegebenenfalls auch parallel dazu - geführt ist, sowie
- Fig. 7: die Überzugwendel der Fig. 6, wobei die Abstände der Windungen dieser Wendel übertrieben groß dargestellt sind.

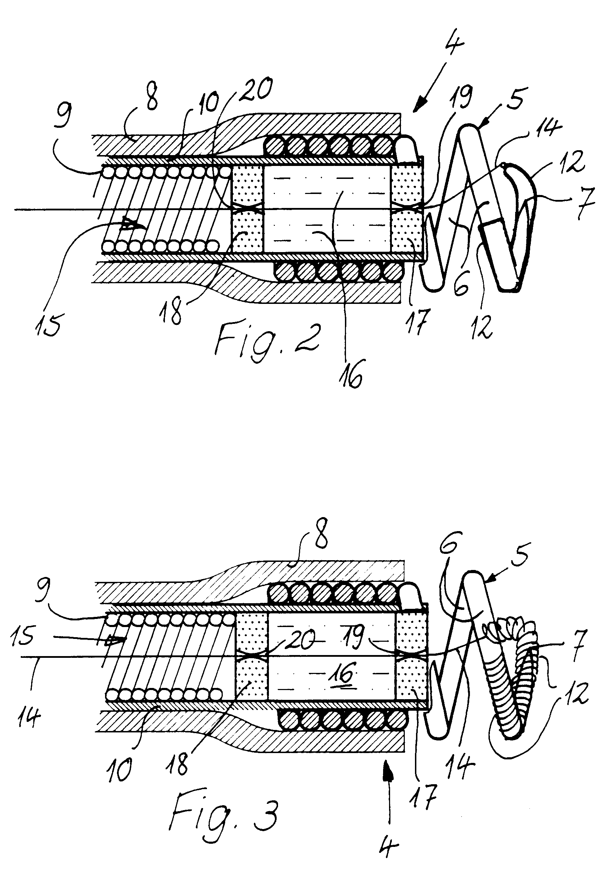

Eine im ganzen mit 1 bezeichnete Herzschrittmacherleitung führt von einem Herzschrittmacher 2 in das Herz 3 und ist dazu transvenös implantiert. Der in den Figuren 2 bis 6 vergrößert dargestellte Elektrodenkopf 4 wird im Inneren des Herzens mit Hilfe einer einschraubbaren Schraubwendel 5 fixiert. Diese aus im Ausführungsbeispiel etwa ein und einer halben Windung 6 bestehende Schraubwendel 5 hat eine scharfe Spitze 7, um das Einschrauben im Inneren des Herzens 3 zu erleichtern. Sie steht gegenüber einem isolierenden Schlauch 8 der Zuleitung 9 der Herzschrittmacherleitung 1 vor und hat einen festen Überstand. Man erkennt deutlich in den Figuren 2 bis 6, daß die aus gewendelten Drahtwindungen bestehende Zuleitung 9 am distalen Ende von einer Hülse 10 umschlossen sind, die Kontakt zu der Schraubwendel 5 herstellen, welche mit weiteren Windungen im Inneren des Schlauches 8 auf dieser Hülse 10 angeordnet ist. Wähend die Einschraubwendel 5 eine relativ große Steigung hat, um ein Einschrauben zu ermögliche, haben ihre kontaktgebenden Windungen im Inneren des Schlauches 8 und an der Außenseite der Hülse 10 keinen Abstand zwischen sich.

Damit die scharfe Spitze 7 beim Implantieren der Herzschrittmacherleitung 1 beziehungsweise der Zuleitung 9 das Gefäß oder die Herzklappen 11 nicht verletzt und sich nicht daran verhaken kann, weist die Schraubwendel 5 einen an ihrer Windung 6 eng anliegenden und wenigstens die Spitze 7 sowie einen Nachbarbereich dazu umkleidenden Überzug 12 auf, der in den verschiedenen Ausführungsbeispielen jeweils unterschiedlich gestaltet ist, aber ein übereinstimmendes Bezugszeichen 12 hat.

In allen Ausführungsformen gemäß den Figuren 2 bis 7 ist vorgesehen, daß die Innen- und Außenkontur dieses Überzuges 12 der Außenkontur des von ihr umkleideten Bereiches der die Schraubwendel 5 bildenden Windung 6 etwa entspricht, d.h. in Gebrauchsstellung stellt der Überzug 12 gewissermaßen ein Stück einer hohlen Wendel in entsprechend gewundener Form dar. Es ist also nicht die gesamte Schraubwendel 5 insgesamt von außen her umschlossen, sondern es ist ein Windungsbereich dieser Wendel und zwar der für Verletzungen oder Verhakungen gefährliche Bereich der Spitze 7 überzogen. In den Ausführungsbeispielen bleibt jeweils ein größerer Teil der Windungen 6 der Schraubwendel 5 frei. Somit ist dieser Überzug 12 der Spitze 7 auch völlig unabhängig und getrennt von dem isolierenden Schlauch 8 der Zuleitung 9.

Wichtig ist, daß an dem Überzug 12 in allen Ausführungsbeispielen ein zum proximalen Ende 13 der Herzschrittmacherleitung 1 führendes Zugelement 14 angreift und daß der Überzug 12 mittels dieses Zugelementes 14 von der Spitze 7 der Schraubwendel 5 abziehbar ist.Bevor nämlich die Schraubwendel 5 mit Hilfe ihrer Windungen 6 und der Spitze 7 in dem Herzgewebe endgültig verankert wird, muß der Überzug 12, der ja die Spitze gegen ein Eindringen in Gewebe schützt, entfernt werden. Mit Hilfe des Zugelementes 14 kann also dieser kappenartig ausgebildete Überzug 12 von dem von ihm beaufschlagten Windungsabschnitt abgezogen werden, so daß die Spitze 7 und die Wendel 5 zum Einschrauben in das Herzgewebe frei werden.

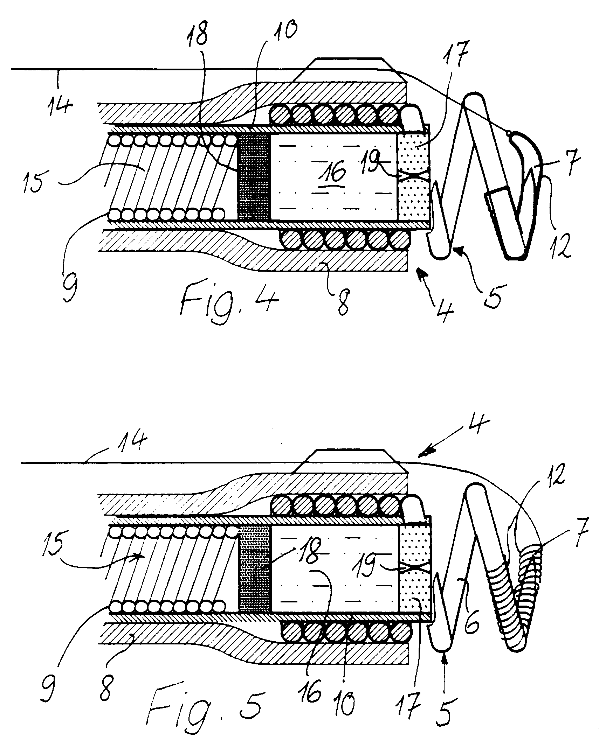

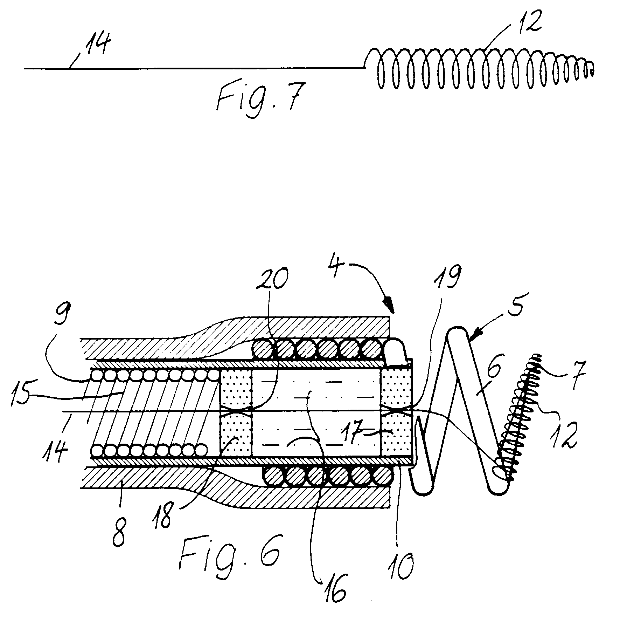

Im Ausführungsbeispiel ist dabei das Zugelement 14 ein Faden oder Draht, der entweder gemäß den Figuren 4 und 5 parallel zu der Herzschrittmacherleitung 1 oder gemäß den Figuren 2, 3 und 6 durch einen in deren Innerem befindlichen Kanal 15 geführt ist. Durch eine entsprechende Zugkraft auf das Zugelement 14 vom distalen Ende 13 aus kann somit der Überzug 12 entweder seitlich an der Herzschrittmacherleitung 1 vorbei oder durch deren Inneres herausgezogen werden, bevor die endgültige Verankerung des Elektrodenkopfes 4 mit Hilfe der Schraubwendel 5 erfolgt.

Einen besonders guten Schutz der Innenseite der Gefäße und der Herzklappen vor der Spitze 7 ergibt sich dabei, wenn gemäß den Figuren 2 bis 5 der Überzug 12 über die Spitze 7 übersteht und gegen den Elektrodenkopf 4 hin umgebogen ist.

Vor allem diese dann unmittelbar vor der Spitze 7 befindliche Umbiegung sorgt für eine genügend große Schutzfläche zum Abschirmen der Spitze 7 gegenüber der Gefäßinnenwand.

Der Überzug 12 kann gemäß den Ausführungsbeispielen unterschiedlich ausgebildet sein. In den Figuren 2 und 4 ist der Überzug 12 ein am distalen Ende geschlossener Schutzschlauch, der die Spitze 7 und wenigstens etwa ein Viertel bis ein Drittel der distalen letzten Windung 6 der Schraubwendel 5, gegebenenfalls auch die halbe oder die gesamte Wendel 5, umschließt. Das Zugelement 14 greift dabei an dem die Spitze umkleidenden Ende dieses kappenförmigen Schlauches an.

Bei den übrigen Ausführungsbeispielen ist der Überzug 12 eine eng gewickelte Wendel aus einem Kunststoff-Filament oder -faden oder aus Metalldraht. Diese Wendel ragt ihrerseits wiederum über die Spitze 7 der Einschraubwendel 5 hinaus und umschließt ebenfalls etwa ein Viertel bis ein Drittel der distalen letzten Windung 6 der Schraubwendel 5, könnte aber auch die halbe oder gar die gesamte Schraubwendel 5 umkleiden. In Fig. 7 ist eine solche Überzug-Wendel vergrößert und vor Gebrauch dargestellt, wobei allerdings der Abstand zwischen den einzelnen Windungen übertrieben groß dargestellt ist und ein guter Schutz vor allem bei einer engeren Anordnung dieser Windungen entsteht. Dabei wird deutlich, daß der Durchmesser des Überzuges 12 - wie auch bei dem schlauchförmigen Überzug - im Bereich der Spitze 7 der Schraubwendel 5 deren Durchmesserverkleinerung folgend abnimmt und bis über diese Spitze 7 reicht und dort einen gegenüber dem Querschnitt der die Schraubwendel 5 bildenden Windung 6 erheblich verminderten Durchmesser hat, so daß ein Zurückschieben dieses Überzuges 12 beim Einführen der Herzschrittmacherleitung 1 durch ein Gefäß vermieden wird.

In diesem Ausführungsbeispiel gemäß den Figuren 6 und 7, aber auch bei der Ausführung gemäß Fig. 3 ist der zum Abziehen des Überzuges 12 dienende Faden oder Draht einstückig mit der Überzug-Wendel verbunden. Somit gibt es keine Probleme mit einer eventuellen Kupplung zwischen dem Zugelement 14 und dem Überzug 12.

Beim Ausführungsbeispiel gemäß Fig. 5 geht dabei der zum Zurückziehen dienende Faden oder Draht von dem im Bereich der Spitze 7 der Schraubwendel 5 liegenden Ende der Überzug-Wendel aus. Man erkennt in den Figuren 3 und 5 deutlich, daß auf diese Weise dieser schon vorgekrümmte Überzug 12 problemlos von dem letzten, der Spitze 7 naheliegenden Bereich der Windung 6 abgezogen werden kann.

Beim Ausführungsbeispiel gemäß den Figuren 6 und 7 greift hingegen das Zugelement 14 an dem proximalen Ende der Überzug-Wendel - in diesem Falle wiederum einstückig - an. Auch dies ergibt ein einfaches Abziehen, weil sich beim Zurückziehen die Windungen des Überzuges 12 mehr und mehr abwickeln können.

In allen Ausführungsbeispielen ist am distalen Ende der Zuleitung 9 eine zu dem Elektrodenkopf 4 gehörende Kammer 16 von der Hülse 10 umschlossen und durch zwei beabstandete Wandungen 17 und 18 abgeteilt, welche ein Medikament enthalten kann, das durch eine distale Eintrittsöffnung 19 in der stirnseitigen Wandung 17 austreten kann. Diese Kammer 16 und ihre Wandungen 17 und 18 dichten den inneren Kanal 15 der Herzschrittmacherleitung 1 gegenden Eintritt von Blut ab. Bei den Ausführungsformen gemäß den Figuren 2, 3 und 6 tritt das Zugelement 14 an dem distalen Ende der Zuleitung 9 durch die enge Öffnung 19 in diese zum Elektrodenkopf 4 gehördende Kammer 16 ein und durch eine weitere Öffnung 20 in der Wandung 18 in das Innere der Zuleitung 9 und in den im Inneren dieser Zuleitung 9 befindlichen Kanal 15 aus. Vor allem die Öffnung 19 erhält somit eine Doppelfunktion, weil sie einerseits das Zugelement 14 führt und aufgrund der Weichheit des Werkstoffes der Wandungen 17 und 18 auch den Durchtritt des Überzuges 12 gestattet, und andererseits das Medikament austreten läßt. Falls kein Medikament mit der Herzschrittmacherleitung 1 appliziert werden soll, kann auf die Wandung 18 mit der Öffnung 20 natürlich verzichtet werden. Auch könnte dann die Hülse 10 statt einer aus weichem und dichtendem Werkstoff bestehenden stirnseitigen Wandung 17 einen den stirnseitigen Abschluß bildenden Boden haben.
Die Herzschrittmacherleitung 1 hat einen isolierenden und ihre Zuleitung 9 überziehenden Schlauch 8 sowie eine zum Fixieren im Inneren des Herzesns 3 dienende und in dessen Gewebe eindrehbare Schraubwendel 5, deren äußerstes und vorderstes Ende spitz und scharf ist und die gegenüber dem Ende des Schlauches 8 einen festen Überstand hat, also keine Axialbewegung gegenüber der Zuleitung 9 ermöglicht. Um diese Schraubwendel 5 und ihre Spitze 7 beim transvenösen Implantieren von der Innenwandung des Gefäßes und auch von den Herzklappen abzuschirmen ist ein die Schraubwendel 5 im Bereich ihrer Spitze 7 umkleidender Überzug 12 vorgesehen, dessen Innen- und Außenkontur der Außenkontur des umkleideten Bereiches der die Schraubwendel 5 bildenden Windung 6 entspricht. An dem Überrzug 12 ist ein zum proximalen Ende 13 der Zuleitung 9 führendes Zugelement 14 vorzugsweise einstückig fixiert, so daß der Überzug 12 mittels dieses Zugelementes 14 von der Spitze 7 der Schraubwendel 5 abgezogen werden kann, wenn diese bis ins Innere des Herzens 3 vorgeschoben ist und kurz bevor sie dann im Herzgewebe verankert wird

## Patentansprüche

1. Herzschrittmacherleitung (1) mit einem isolierenden Schlauch (8) und mit einer zum Fixieren im Inneren des Herzens (3) dienenden, in dieses einschraubbaren Schraubwendel (5), deren äußerstes und vorderstes Ende (7) spitz und scharf ist, die gegenüber dem Schlauchende einen festen Überstand hat, und eine abziehbare Schutzvorrichtung aufweist, **dadurch gekennzeichnet,** daß die Schraubwendel (5) einen an ihrer Windung (6) anliegenden und wenigstens die Spitze (7) umkleidenden Überzug (12) aufweist, dessen Innen- und Außenkontur der Außenkontur des von ihr umkleideten Bereiches der die Schraubwendel (5) bildenden Windung (6) etwa entspricht, daß an dem Überzug (12) ein zum proximalen Ende (13) der Herzschrittmacherleitung (1) führendes Zugelement (14) angreift und daß der Überzug (12) mittels des Zugelementes (14) von der Spitze (7) der Schraubwendel (5) abziehbar ist.

2. Herzschrittmacherleitung nach Anspruch 1 , dadurch gekennzeichnet, daß der Überzug (12) kappenartig ausgebildet und in Gebrauchsstellung als Hohlwindung oder Windungsabschnitt entsprechend der Krümmung der Windung der Schraubwendel (5) geformt ist.

3. Herzschrittmacherleitung nach Anspruch 1 oder 2, dadurch gekennzeichnet, daß das Zugelement (14) ein Faden oder Draht ist, der prallel zu der Herzschrittmacherleitung (1) beziehungsweise deren Zuleitung (9) oder durch einen in deren Innerem befindlichen Kanal (15) geführt ist.

4. Herzschrittmacherleitung nach einem der Ansprüche 1 bis 3, dadurch gekennzeichnet, daß der Überzug (12) ein am distalen Ende geschlossener Schutzschlauch ist, der die Spitze (7) und wenigstens etwa ein Viertel bis ein Drittel der distalen letzten Windung (6) der Schraubwendel (5), gegebenenfalls die halbe oder die gesamte Schraubwendel (5), umschließt und daß das Zugelement (14) vorzugsweise an dem die Spitze (7) umkleidenden Ende des Schlauches angreift oder befestigt ist.

5. Herzschrittmacherleitung nach einem der Ansprüche 1 bis 3, dadurch gekennzeichnet, daß der Überzug (12) eine eng gewickelte Wendel aus einem Kunststoff-Filament oder aus Metalldraht ist, die über die Spitze (7) der Einschraubwendel (5) ragt.

6. Herzschrittmacherleitung nach einem der Ansprüche 1 bis 5, dadurch gekennzeichnet, daß der Durchmesser des Überzuges (12) im Bereich der Spitze (7) der Schraubwendel (5) deren Durchmesserverkleinerung gegen die Spitze (7) hin folgend abnimmt und bis über diese Spitze (7) reicht und dort einen gegenüber dem Durchmesser oder Querschnitt der die Schraubwendel (5) bildenden Windung (6) erheblich verminderten Durchmesser oder lichten Querschnitt hat.

7. Herzschrittmacherleitung nach einem der Ansprüche 1 bis 6, dadurch gekennzeichnet, daß der zum Abziehen des Überzuges (12) dienende Faden oder Draht einstückig mit dem als Schlauch oder Wendel ausgebildeten Überzug (12) verbunden ist.

8. Herzschrittmacherleitung nach einem der Ansprüche 1 bis 3 oder 5 bis 7, dadurch gekennzeichnet, daß der zum Zurückziehen dienende Faden oder Draht von dem im Bereich der Spitze (7) der Schraubwendel (5) liegenden Ende der Überzug-Wendel ausgeht.

9. Herzschrittmacherleitung nach einem der Ansprüche 1 bis 3 oder 5 bis 7, dadurch gekennzeichnet, daß das Zugelement (14) an dem proximalen Ende der Überzug-Wendel angreift oder einstückig damit verbunden ist.

10. Herzschrittmacherleitung nach einem der Ansprüche 1 bis 9, dadurch gekennzeichnet, daß das Zugelement (14) an dem distalen Ende der Zuleitung (9) durch eine enge Öffnung (19) in eine zu dem Elektrodenkopf (4) gehörende Kammer (16) eintritt und radial geführt ist und durch eine weitere, diese Kammer (16) begrenzende Wandung (18) und eine Öffnung (20) in das Innere der Zuleitung (9) und in den im Inneren dieser Zuleitung (9) befindlichen Kanal (15) austritt.

11. Herzschrittmacherleitung nach einem der Ansprüche 1 bis 10, dadurch gekennzeichnet, daß die im Elektrodenkopf (4) befindliche Kammer (16) ein Medikament enthält, welches durch die distale Öffnung (19) für das Zugelement (14) allmählich austritt.

12. Herzschrittmacherleitung nach einem der Ansprüche 1 bis 11, dadurch gekennzeichnet, daß der Überzug (12) über die Spitze (7) übersteht und dieser Überstand vorzugsweise gegen den Elektrodenkopf (4) hin umgebogen ist.

## Claims

1. A cardiac pacemaker lead (1) having a flexible insulating tubing (8) and further having a helix (5) which serves for fixation inside the heart (3) and is adapted to be screwed into the same, the outermost and foremost end (7) of said helix being pointed and sharp: being at a fixed distance from the tubing end and having an extractable protector,
**characterized in that** the helix (5) has adjacent its convolution (6) a jacket (12) sheathing at least the tip (7), the internal and external contour of said jacket conforming generally to the outer contour of the area it sheathes of the convolution (6) composing the helix (5), that the jacket (12) is engaged by a draw element (14) leading to the proximal end (13) of the cardiac pacemaker lead (1) and that the jacket (12) is extractable from the tip (7) of the helix (5) by means of the draw element (5).

2. A cardiac pacemaker lead as claimed in claim 1, characterized in that the jacket (12) is of cap-like configuration and in the position of use is shaped as a hollow convolution or convolution portion so as to conform with the curvature of the convolution of the helix (5)

3. A cardiac pacemaker lead as claimed in claim 1 or claim 2, characterized in that the draw element (14) is a thread or wire conducted parallel to the cardiac pacemaker lead (1) or supply lead (9) thereof or through a passageway (15) in the interior thereof.

4. A cardiac pacemaker lead as claimed in any one of claims 1 to 3, characterized in that the jacket (12) is a protective tubing which is closed at the distal end and surrounds the tip (7) and at least about a quarter to a third of the distal, last convolution (6) of the helix (5), possibly half or the entire helix (5), and that the draw element (14) engages or is attached preferably to the tubing end sheathing the tip (7).

5. a cardiac pacemaker lead as claimed in any one of claims 1 to 3, characterized in that the jacket (12) is a tight spiral of a filamentary plastic material or metallic wire, projecting beyond the tip (7) of the helix (5).

6. A cardiac pacemaker lead as claimed in any one of claims 1 to 4, characterized in that in the area of the tip (7) of the helix (5) the diameter of the jacket (12) diminishes following the diminution of diameter of said helix towards the tip (7) and extends beyond said tip (7) and there has a substantially reduced diameter or inside cross section compared to the diameter or cross section of the convolution (6) composing the helix (5).

7. A cardiac pacemaker lead as claimed in any one of claims 1 to 6, characterized in that the thread or wire serving for extracting the jacket (12) is integrally connected to the jacket (12) taking the form of a tube or spiral.

8. A cardiac pacemaker lead as claimed in any one of claims 1 to 3 or 5 to 7, characterized in that the thread or wire serving for extraction departs from that end of the jacket spiral which is located in the area of the tip (7) of the helix (5).

9. A cardiac pacemaker lead as claimed in any one of claims 1 to 3 or 5 to 7, characterized in that the draw element (14) engages or is integrally connected to the proximal end of the jacket spiral.

10. A cardiac pacemaker lead as claimed in any one of claims 1 to 9, characterized in that the draw element (14) enters the distal end of the supply lead (9) and is radially guided through a narrow opening (19) into a chamber (16) belonging to the electrode head (4), and exits through a further wall (18) defining said chamber (16) and through an opening (20) into the interior of the supply lead (9) and into the passageway (15) located inside said supply lead (9).

11. A cardiac pacemaker lead as claimed in any one of claims 1 to 10, characterized in that the chamber (16) situated in the electrode head (4) contains a medicament gradually dispensed through a distal opening (19) for the draw element (14).

12. A cardiac pacemaker lead as claimed in any one of claims 1 to 11, characterized in that the jacket (12) projects beyond the tip (7) and said projection is preferably bent over towards the electrode head (4).

## Revendications

1. Conduit (1) de stimulateur cardiaque, avec un tuyau souple isolant (8) et avec une hélice (5), qui sert à la fixation à l'intérieur du coeur (3), peut être vissée dans ce dernier, possède une extrémité (7) la plus extérieure et la plus avant qui est pointue et acérée, présente un porte-à-faux fixe par rapport à l'extrémité du tuyau souple et possède un dispositif de protection retirable, **caractérisé** en ce que l'hélice (5) présente une gaine (12) appliquée contre sa spire (6), enveloppant au moins la pointe (7) et dont le contour intérieur et extérieur correspond approximativement au contour extérieur de la région, enveloppée par cette gaine, de la spire (6) constituant l'hélice (5), en ce qu'un élément de traction (14) menant à l'extrémité proximale (13) du conduit (1) de stimulateur cardiaque agit sur la gaine (12), et en ce que la gaine (12) peut être retirée de la pointe (7) de l'hélice (5) au moyen de l'élément de traction (14).

2. Conduit de stimulateur cardiaque selon la revendication 1, **caractérisé** en ce que la gaine (12) est configurée à la manière d'un capuchon et est façonnée, en position d'utilisation, en une spire creuse ou tronçon de spire conformément à la courbure de la spire de l'hélice (5).

3. Conduit de stimulateur cardiaque selon la revendication 1 ou 2, **caractérisé** en ce que l'élément de traction (14) est un fil textile ou un fil métallique, qui est guidé parallèlement au conduit (1) de stimulateur cardiaque ou encore à sa ligne d'alimentation (9), ou à travers un canal (15) se trouvant à l'intérieur de cette dernière.

4. Conduit de stimulateur cardiaque selon l'une quelconque des revendications 1 à 3, **caractérisé** en ce que la gaine (12) est un tuyau souple de protection qui est fermé à l'extrémité distale et qui entoure la pointe (7) et au moins environ un quart à un tiers de la dernière spire distale (6) de l'hélice (5), voire la moitié ou la totalité de l'hélice (5), et en ce que l'élément de traction (14), de préférence, agit ou est fixé à l'extrémité du tuyau souple qui enveloppe la pointe (7).

5. Conduit de stimulateur cardiaque selon l'une quelconque des revendications 1 à 3, **caractérisé** en ce que la gaine (12) est une spire étroitement enroulée constituée d'un filament de matière synthétique ou de fil métallique, qui dépasse de la pointe (7) de l'hélice vissable (5).

6. Conduit de stimulateur cardiaque selon l'une quelconque des revendications 1 à 5, **caractérisé** en ce que le diamètre de la gaine (12) dans la région de la pointe (7) de l'hélice (5) diminue en suivant la diminution de diamètre de cette dernière en direction de la pointe (7), et s'étend jusqu'à cette pointe (7) et y possède un diamètre ou une section intérieure considérablement réduit(e) par rapport au diamètre ou à la section de la spire (6) constituant l'hélice (5).

7. Conduit de stimulateur cardiaque selon l'une quelconque des revendications 1 à 6, **caractérisé** en ce que le fil textile ou métallique servant à retirer la gaine (12) est relié d'un seul tenant à la gaine (12) réalisée sous forme de tuyau souple ou d'hélice.

8. Conduit de stimulateur cardiaque selon l'une quelconque des revendications 1 à 3 ou 5 à 7, **caractérisé** en ce que le fil textile ou métallique servant à la rétraction part de l'extrémité de la gaine en hélice qui est située dans la région de la pointe (7) de l'hélice (5).

9. Conduit de stimulateur cardiaque selon l'une quelconque des revendications 1 à 3 ou 5 à 7, **caractérisé** en ce que l'élément de traction (14) agit à l'extrémité proximale de la gaine en hélice ou est relié d'un seul tenant à cette dernière.

10. Conduit de stimulateur cardiaque selon l'une quelconque des revendications 1 à 9, **caractérisé** en ce que l'élément de traction (14), à l'extrémité distale de la ligne d'alimentation (9), pénètre par une étroite ouverture (19) dans une chambre (16) faisant partie de la tête d'électrode (4), est guidé radialement et, en traversant par une ouverture (20) une autre paroi (18) délimitant cette chambre (16), sort à l'intérieur de la ligne d'alimentation (9) et dans le canal (15) qui se trouve à l'intérieur de cette ligne d'alimentation (9).

11. Conduit de stimulateur cardiaque selon l'une quelconque des revendications 1 à 10, **caractérisé** en ce que la chambre (16) qui se trouve dans la tête d'électrode (4) contient un médicament qui s'échappe progressivement par l'ouverture distale (19) pour l'élément de traction (14).

12. Conduit de stimulateur cardiaque selon l'une quelconque des revendications 1 à 11, **caractérizé** en ce que la gaine (12) dépasse de la pointe (7), et cette partie dépassante est de préférence recourbée vers la tête d'électrode (4).
